# EUROPEAN PATENT APPLICATION

(11) **EP 2 289 528 A1**
(43) Date of publication of application: **02.03.2011**
(21) Application number: 09166020.9
(22) Date of filing: 21.07.2009
(51) Int. Cl.: A61K 36/67, A61P 25/00

(54) **Novel nutraceutical compositions containing black pepper or its constituents improving mental performance**

(71) Applicant: DSM IP Assets B.V., 6411 TE Heerlen (NL)
(72) Inventor: The designation of the inventor has not yet been filed
(74) Representative: Mezger, Wolfgang

(57) **Abstract**

The invention relates to a novel nutraceutical composition piperine, piperlongumine, or a plant extract containing piperine, piperlongumine as active ingredient(s). The term "nutraceutical" as used herein denotes usefulness in nutritional, pharmaceutical and veterinary fields of application. The compositions are useful for improvement of cognitive functions and psycho-social status, such as learning, memory and alertness, psychotic stability and maintenance.

## Description

### FIELD OF THE INVENTION

The present invention relates to a novel nutraceutical composition or food additive comprising piperine, piperlongumine or a plant extract comprising piperine, and/or piperlongumine as active ingredient(s) to improve cognitive functions such as learning, memory and alertness as well as relieving psychosocial pressure.

### BACKGROUND OF THE INVENTION

Memory, learning and alertness rely on neuronal circuits in the midbrain, especially in the hippocampus where information is processed and memory is consolidated. Mental performance and learning are dependent on synaptic plasticity; i.e. strengthening neuronal connections by the recruitment of new receptors, formation of new synapses and eventually the generation of new neuronal connections.

The formation of (long-term) memory and the efficient functioning of the brain depend on synthesis of new proteins for the reinforcement of communicative strength between neurons. The production of new proteins devoted to synapse reinforcement is triggered by chemical and electrical signals within neurons.

Long term potentiation (LTP) is the term used to describe the long-lasting enhancement of synaptic transmission (hours *in vitro,* days or weeks *in vivo*) which occurs at particular synapses within the central nervous system (CNS) following a short, conditioning, burst of presynaptic electrical stimulation (approximately 100 Hz for 1 second). This phenomenon is widely considered to be one of the major mechanisms by which memories are formed and stored in the brain. LTP has been observed both *in vitro* and in living animals. Under experimental conditions, applying a series of short, high-frequency electric stimuli to a synapse can potentiate the strength of the chemical synapse for minutes to hours. Most importantly, LTP contributes to synaptic plasticity in living animals, providing the foundation for a highly adaptable nervous system.

Two different receptor types are primarily involved in the process of LTP, namely the N-methyl-D-aspartate (NMDA) receptor complex and the alpha-amino-3-hydroxy-5-methyl-4-isoxazole propionic acid (AMPA) receptor. During LTP, the major excitatory neurotransmitter, glutamate, is released from the presynaptic neuron, binds to and activates the AMPA receptor on the postsynaptic membrane, leading to its depolarisation. At resting membrane potentials, the NMDA receptor channel is blocked by magnesium ions, but depolarisation of the postsynaptic membrane removes this block, enabling NMDA receptor activation and subsequent entry of calcium into the cell. This rise in intracellular calcium is believed to activate protein kinases, leading to gene transcription and the construction of reinforcing proteins (Niehoff (2005), The Language of Life: How Cells Communicate in Health and Disease, 210-223) and resulting in enhanced sensitivity of the AMPA receptor, thus further facilitating neurotransmission and maintenance of LTP. NMDA receptors are composed of assemblies of NR1- and NR2- subunits; the glutamate binding domain is formed at the junction of these subunits. In addition to glutamate, the NMDA receptor requires a co-agonist, glycine, in order to modulate receptor function. The glycine binding site is found on the NR1 subunit, while the NR2 subunit possesses a binding site for polyamines, regulatory molecules that modulate the functioning of the NMDA receptor.

The amino acid glycine is thus known to act as a positive allosteric modulator and obligatory co-agonist with glutamate at the NMDA receptor complex (Danysz 1998, Pharmacol. Rev., 50 (4), 597-664). Glycine transporters (GlyT) play an important role in the termination of postsynaptic glycinergic actions and maintenance of low extracellular glycine concentrations by reuptake of glycine into presynaptic nerve terminals or glial cells. The termination of the action of glycine is therefore largely mediated by rapid reuptake. Two glycine transporters, GlyT1 and GlyT2, are known and are characterized by 12 putative transmembrane regions, while three variants of GlyT1 (GlyT1a, b, and c) encoded from the same gene have been identified (Borowsky and Hoffman (1998), J. Biol. Chem., 273 (44), 29077-29085).

GlyT1 is the only sodium chloride-dependent glycine transporter in the forebrain, where it is co-expressed with the NMDA receptor. At this site, GlyT1 is thought to be responsible for controlling extracellular levels of glycine at the synapse (López-Corcuera (2001), Mol. Membr. Biol., 18 (1), 13-20), resulting in modulation of NMDA receptor function. Indeed, in the presence of the selective GlyT1 antagonist *N*-[3-(4'-fluorophenyl)-3-(4'-phenylphenoxy)] propylsarcosine (NFPS), enhanced NMDA receptor responses in CA1 pyramidal cells were observed upon Schaffer collateral stimulation in both mouse and rat hippocampal slice preparations (Bergeron, et al (1998), Proc. Natl. Acad. Sci. USA, 95 (26),15730-15734). *In vivo,* systemic administration of NFPS increased LTP in the dentate gyrus and enhanced prepulse inhibition of the acoustic startle response in adult mice, indicating that inhibition of GlyT1 affects NMDA receptor function in a behaviourally-relevant way (Kinney, et al (2003), J. Neurosci., 23 (20), 7586-7591). Such data highlight the potential usefulness of compounds which can enhance NMDA receptor synaptic function by elevating extracellular levels of glycine in the local microenvironment of synaptic NMDA receptors, for the prevention of psychotic syndromes and for maintaining or boosting physiological cognitive functions, such as memory and learning, in normal individuals.

There is an increasing interest in the development of compounds, as well as nutraceutical compositions, that may be used to improve learning, memory and alertness, in both elderly and young people, individuals who need especially high memory and attention in their daily work, including students, construction workers, drivers, pilots, physicians, salespeople, executives, housewives, "high performance professionals" and people who are under mental or daily stress as well as persons who are prone to psychiatric instability, such as schizophrenia.

Thus, a compound or nutraceutical composition which enhances NMDA receptor function and enables improvements in learning, memory and alertness would be highly desirable.

JP2008-214338A teaches a combination of alpha-lipoic acid, ginko leaf extract, creatine, carnitine, black pepper extract and L-cysteine as a brain-functional improving agent, particularly for preventing and treating forgetfulness and dementia.

US 7,371,389 (Arbonne Intl.) teaches a nutritional product containing a combination of vitamins, herbs, enzymes and "superfoods" and which includes black pepper powder. This is used to enhance energy and nutrition.

US 5,972,382 (Reexamined) (Sabinsa Corp.) teaches the use of compositions containing at least 98% pure piperine. These compositions increase the gastrointestinal absorption and systemic bioavailability of other nutrient compounds, such as vitamins, herbal extracts, amino acids, minerals and antioxidants.

WO 2002/052955 discloses the use of a composition containing a mixture of a) lipids, b) terpenes c) 1-piperoylpiperidine, preferably from a black pepper extract, and d) polycosanol. One of the claimed uses, among many others, is the improvement of higher brain functions, ability to efficiently use and create energy in addition to beneficial effects on preventing and treating aging processes and related conditions.

The extract of the present invention differs from that disclosed in above applications which are aqueous extracts.

### DETAILED DESCRIPTION OF THE INVENTION

It has been found, in accordance with this invention, that piperine and piperlongumine are activators of hippocampal function, as evidenced by their ability to induce LTP. They can work either by inhibiting the glycine transporter, GlyT1, thus inhibiting reuptake of glycine, or by activation of another pathway, or by both mechanisms. These biological activities are important in memory formation and memory consolidation, thus these compounds are useful in enhancing cognitive functions.

In particular, it has been found in accordance with the invention, that piperine and plant extracts which contain piperine, have the ability to inhibit glycine reuptake by inhibiting the glycine transporter, GlyT1. The resulting increase in extracellular glycine levels leads to additional activation of NMDA receptors, which is the first step to inducing transcriptional activation of a number of genes and subsequently to induce LTP, the main cellular mechanism involved in memory formation and memory consolidation.

It has also been found that piperlongumine and plant extracts containing piperlongumine enables induction of LTP through a different mechanism than does piperine. As both processes have the same biological benefits, i.e. they both facilitate hippocampal functioning leading to enhanced cognitive functioning, another aspect of this invention is the use of these two active ingredients together to enhance cognitive functions.

Therefore one aspect of the invention is a novel nutraceutical composition, comprising piperine and/or piperlongumine to enhance cognitive functions. These compounds may be preferably present in a plant extract, and preferably an extract from the fruit of black pepper.

It has been found, in accordance with this invention, that an extract of black pepper has the ability to induce LTP, which is important in memory formation and memory consolidation. As such, the black pepper fruit extract is useful in enhancing cognitive functions. Therefore one aspect of the invention is a novel nutraceutical composition, comprising black pepper whole-fruit extract to enhance cognitive functions.

### BRIEF DESCRIPTION OF THE FIGURES

FIGURE 1 shows the dose-response curves of piperine in the GlyT1 inhibition assay.
FIGURE 2 shows the dose response curves of a black pepper extract in the GlyT1 inhibition assay.

### Plant Extracts

Piperine and/or piperlonumine can either be synthetically produced using known methods, or they can be extracted from natural sources such as plants using known extraction procedures.

There are a number of plant species which bear fruit which contain piperine and/or piperlongumine and may be the source of the plant extract. Preferably the plant is a member of the species *Piper.* Suitable plants include: *Piper nigrum, P. longum, P. tuberculatum, P. hancei, P. hispidum, P. retrofactum, P. attenuatum, P. genueense, P. chaba, P. aurantiacum, P. auritum, P. cubeba, P. peltatum, P.umbellatum,* and *P*. *mystheticum. P. nigrum* is preferred, as it contains both compounds, but the source of the extract may be any plant known to contain either compound.

Generally, the pepper extract should contain at least about 20% piperine, and from about 2% piperlongumine, and more preferably from about 30% piperine and at least about 3% piperlongumine.

As used throughout the specification and claims, the term "black pepper extract" is intended to be used broadly, and can encompass plant extracts made by conventional means, such as steam distillation, supercritical CO₂ (SF-CO₂) extractions, water-based extractions, nitrous oxide extractions, alcohol-based extractions, or organic solvent-based extractions, such as ethyl acetate, propane, acetone, optionally modified with modifiers such as ethanol. Preferred extracts include SF-CO₂, hydro-/steam distillations, ethanol, and ethyl acetate extracts. As the extract is intended for human and animal consumption, the extract should be one which is approved by regulatory agencies.

The only critical parameters regarding the black pepper extract are:
1) It should be acceptable for use in a nutraceutical composition for animal or human consumption. Thus the solvent to be employed for its preparation should be approved by the various regulatory agencies for the intended use. Therefore, preferred extraction procedures are steam distillation, SF-CO₂, C₂₋₄alcohol extractions, n-hexane and ethyl acetate extractions.
2) It should contain a sufficient amount of the active piperine, piperlongumine, or any combinations thereof so that the desired effects, such as cognition improvment and/or improvement in psycho-social status are achieved, i.e. the subject exhibits an improved memory function or can cope with stressful situations in a more productive fashion.

As used throughout the specification and claims, the terms "cognitive function-improving amount" and "psycho-social status -improving amount" are intended to convey that cognition improvement and/or improvement in psycho-social status are achieved, i.e. the subject exhibits an improved memory function or can cope with stressful situations in a more productive fashion. The amount of improvement of the various states can be assessed using standardized psychological assessment tests.

Black pepper extracts typically contain other compounds which may also be bioactive, and/or increase the bioavailability of the active components. The amounts in which they are present in the black pepper extract will vary, based on a number of factors, including: the plant species used, the growing conditions of the plant, and, of course, the processes used to prepare the extract. A typical pepper extract prepared using supercritical CO₂ methods will contain piperine, pinene, sabinene, limonene, caryophyllene, phellandrene, piperlonguminine.

### Benefits of black pepper extract for mental states

As previously described, the basis of memory, learning and mental stability is LTP, or the strengthening of neuronal connections, which occurs via activation of AMPA and NMDA receptors within the brain, particularly in the hippocampus.

As glycine reuptake inhibitors through their activity at GlyT1, piperine and plant extracts containing them, enable accumulation of glycine in the vicinity of the NMDA receptor, thus activating it and ultimately resulting in the induction of LTP, the main cellular mechanism involved in memory formation and memory consolidation.

Moreover, piperlongumine induces activation of the LTP leading to LTP induction, and is likewise beneficial in improving memory functions. Thus, piperine and piperlongumine, and extracts containing either or both, can activate hippocampal functions and improve memory formation and consolidation, as well as improve mental health.

### Conditions improved by this invention:

In the context of this invention "treatment" also encompasses co-treatment as well as prevention. "Prevention" includes delaying the onset of a condition or symptom, and lessening the severity of the condition or symptom when it does appear, as well as early intervention of a condition or symptom.

Throughout this specification and claims, the term "improved cognitive function" is meant to refer to the conditions of supporting and maintaining cognitive wellness and balance, such as:
- Enhanced learning, including:
   o language processing
   o problem solving
   o intellectual functioning
- Ability to cope with psychosocial burdens
- Enhanced attention and concentration
- Enhanced memory and the capacity for remembering, especially short-term memory
- Enhanced mental energy, alertness and mental vigilance, reduction of mental fatigue
- Stabilization of mental status including:
   o Relieving post-partum conditions
   o Relieving psychological burden due to separation of partners, children, death of beloved people or marital problems
   o Relieving problems associated with change of domicile, work or similar events
   o Relieving stressful conditions following a traffic accident or other negative social pressure
- Stress relief, including:
   o treatment, prevention and alleviation of symptoms related to work overload, exhaustion and/or "bum out"
   o increased resistance or tolerance to stress
   o favouring and facilitating relaxation in normal healthy individuals
- "Condition improvement", including:
   o reducing irritability and tiredness
   o reducing, preventing or alleviating physical and mental fatigue
   o promoting good-quality sleep, that is to act against insomnia and sleep disorders and to increase energy in more general terms, in diseased or normal healthy individuals.

In a preferred aspect of the present invention the compositions may be used as nutritional supplements, particularly for people who may feel a special need for enhanced cognitive function and/or psychosocial support. A non-exhaustive list of people who would benefit from enhanced cognitive function would include:
o elderly people,
o students or persons who are preparing for exams,
o children who are engaged in a great deal of learning, i.e. infants, toddlers, pre-school children and school children,
o construction workers, or those operating potentially dangerous machinery,
o truck drivers, pilots, train drivers, or other transportation professionals,
o air traffic controllers,
o salespeople, executives, and other "high performance professionals"
o police officers and military personnel,
o housewives,
or for anyone exposed to high amounts of stress in their daily work or who needs especially high attention/concentration/ high mental and psychological performance in their daily work, such as those participating in sports, chess players, golfers, professional performers (actors, musicians and the like).

To achieve these improvements, administration over several days (for example at least 6 or 10 days) is recommended, and administration daily for several weeks is generally preferred.

Aside from applications for humans, the compositions of this invention have additional uses in the veterinary world. Animals which can benefit from enhanced cognitive function include those animals trained for specific purposes, including guide dogs, police and military dogs, and dogs trained to detect survivors from earthquakes, avalanche, landslides, and other environmental or military catastrophes, and the like. Further, virtually any young animal in its growth, development, education and training phase will benefit from a supplementation to enhance learning capabilities.

In addition, animals which are subject to stressful conditions can benefit from this invention. Such conditions occur, for example, after capture or transport or may be due to housing conditions (such as change of domicile or owner), when the animals develop analogous disorders and are distressed or aggressive, or display stereotypic behavior, or anxiety and obsessive-compulsive behavior. Animals which are subject to stress would also include those which are racing animals (e.g. dogs, horses, camels), or used in various sports, performing animals (such as circus animals and those appearing on stage, television or in the movies) and horses which perform dressage and other highly disciplined routines.

Preferred "animals" are pets or companion animals and farm animals. Examples of pets are dogs, cats, birds, aquarium fish, guinea pigs, (jack) rabbits, hares and ferrets. Examples of farm animals are aquaculture fish, pigs, horses, ruminants (cattle, sheep and goats) and poultry.

### Nutraceutical uses/formulations/dosages

The term "nutraceutical" as used herein denotes usefulness in both nutritional and pharmaceutical fields of application. Thus, novel nutraceutical compositions can be used as supplements to food and beverages and as pharmaceutical formulations for enteral or parenteral application which may be solid formulations, such as capsules or tablets, or liquid formulations, such as solutions or suspensions.

The nutraceutical compositions according to the present invention may further contain protective hydrocolloids (such as gums, proteins, modified starches), binders, film-forming agents, encapsulating agents/materials, wall/shell materials, matrix compounds, coatings, emulsifiers, surface active agents, solubilising agents (oils, fats, waxes, lecithins etc.), adsorbents, carriers, fillers, co-compounds, dispersing agents, wetting agents, processing aids (solvents), flowing agents, taste-masking agents, weighting agents, jellyfying agents, gel-forming agents, antioxidants and antimicrobials.

Moreover, a multi-vitamin and mineral supplement may be added to nutraceutical compositions of the present invention to obtain an adequate amount of an essential nutrient, which is missing in some diets. The multi-vitamin and mineral supplement may also be useful for disease prevention and protection against nutritional losses and deficiencies due to lifestyle patterns.

The nutraceutical compositions according to the present invention may be in any galenic form that is suitable for administering to the body, especially in any form that is conventional for oral administration, e.g. in solid forms such as (additives/supplements for) food or feed, food or feed premix, fortified food or feed, tablets, pills, granules, dragées, capsules and effervescent formulations, such as powders and tablets, or in liquid forms, such as solutions, emulsions or suspensions as e.g. beverages, pastes and oily suspensions. The pastes may be incorporated in hard or soft shell capsules, whereby the capsules feature e.g. a matrix of (fish, swine, poultry, cow) gelatine, plant proteins or ligninsulfonate. Examples for other application forms are those for transdermal, parenteral or injectable administration. The dietary and pharmaceutical compositions may be in the form of controlled (delayed) release formulations.

Examples of food are dairy products including, for example, margarines, spreads, butter, cheese, yoghurts or milk-drinks. Examples of fortified food are cereal bars, bakery items, such as cakes and cookies, and potato chips or crisps. Beverages encompass non-alcoholic and alcoholic drinks as well as liquid preparations to be added to drinking water and liquid food. Non-alcoholic drinks are e.g. soft drinks, sports drinks, fruit juices, lemonades, teas and milk-based drinks. Liquid foods are e.g. soups and dairy products. The nutraceutical composition containing piperine, piperlongumine and/or a black pepper extract or other constituents thereof may be added to a soft drink, an energy bar, cereal, chocolate or a candy, such that a consumer ingests a cognitive-function improving amount of containing piperine, piperlongumine and/or an enriched black pepper extract, ranging from about 10 to 1000 mg per daily serving, preferably from about 50 to 750 mg per daily serving, or more preferably from about 100 to 500 mg per daily serving.

If the nutraceutical composition is a pharmaceutical formulation the composition further contains pharmaceutically acceptable excipients, diluents or adjuvants. Standard techniques may be used for their formulation, as e.g. disclosed in Remington's Pharmaceutical Sciences, 20th edition Williams & Wilkins, PA, USA. For oral administration, tablets and capsules are preferably used which contain a suitable binding agent, e.g. gelatine or polyvinyl pyrrolidone, a suitable filler, e.g. lactose or starch, a suitable lubricant, e.g. magnesium stearate, and optionally further additives. Daily dosages are substantially the same as those above for food formulations, but for ease of administration, may be divided into smaller doses per administration unit, and multiple administration units (such as 1-4 capsules) may be taken daily.

Preferred are plant extracts providing a daily dosage of 0.01-1000 mg, more preferably 5 - 750 mg, and even more preferably 50-500 mg of piperine and/or piperlongumine. For each ingredient separately, the preferred daily dosage is about 2.5-500 mg, preferably 5-375mg, and even more preferably 25-250 mg.

For animals excluding humans a suitable daily dosage of piperine, piperlongumine or an enriched black pepper extract, for the purposes of the present invention may be within the range from 0.001 mg per kg body weight to about 1000 mg per kg body weight per day. More preferred is a daily dosage of about 0.5 mg to about 500 mg per kg body weight, and especially preferred is a daily dosage of about 1 mg to 100 mg per kg body weight.

The following non-limiting Examples are presented to better illustrate the invention.

### Example 1

### Black pepper extract

The black pepper extract was an SFCO2 extract commercially available from Flavex, Rehlingen, Germany.

### Example 2

### Inhibition of glycine transporter 1 in a cellular assay

CHO cells stably expressing the human glycine transporter 1b cDNA (GlyT1) were routinely grown in Dulbecco's Modified Eagle's Medium (purchased from Invitrogen, Carlsbad, USA) containing 10% dialyzed fetal calf serum, penicillin, streptomycin, proline and the antibiotic G418. Cells were harvested by trypsinisation one day prior to the assay and were seeded in the above mentioned medium. Immediately prior to the assay, the medium was replaced by uptake buffer containing 150 mM NaCl, 1 mM CaCl₂, 2.5 mM KCl, 2.5 mM MgCl₂, 10 mM Glucose and 10 mM N-2-hydroxyethylpiperazine-N'-2-ethanesulfonic acid ("Hepes" buffer).

Glycine uptake into the cells was determined by addition of 60 nM radio-labelled [³H] glycine (Amersham Biosciences GE Healthcare, Slough, UK) and incubation for 30 minutes at room temperature. Following removal of unincorporated label by gentle washing three times with the above buffer, incorporated glycine was quantified by liquid scintillation counting.

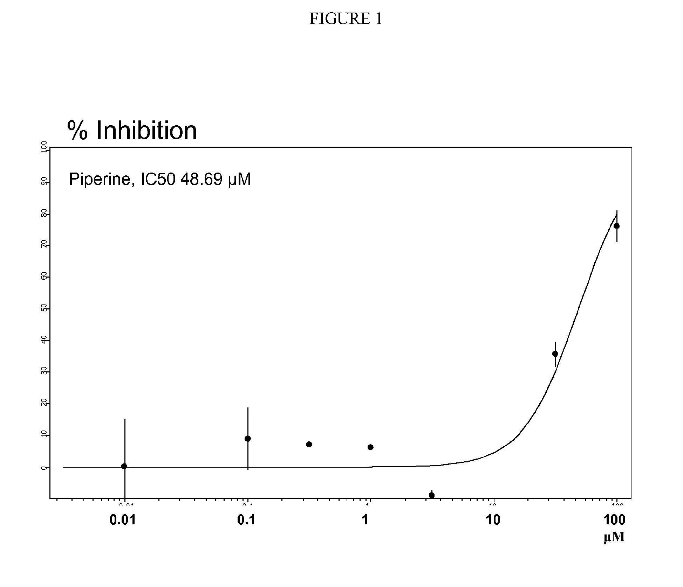

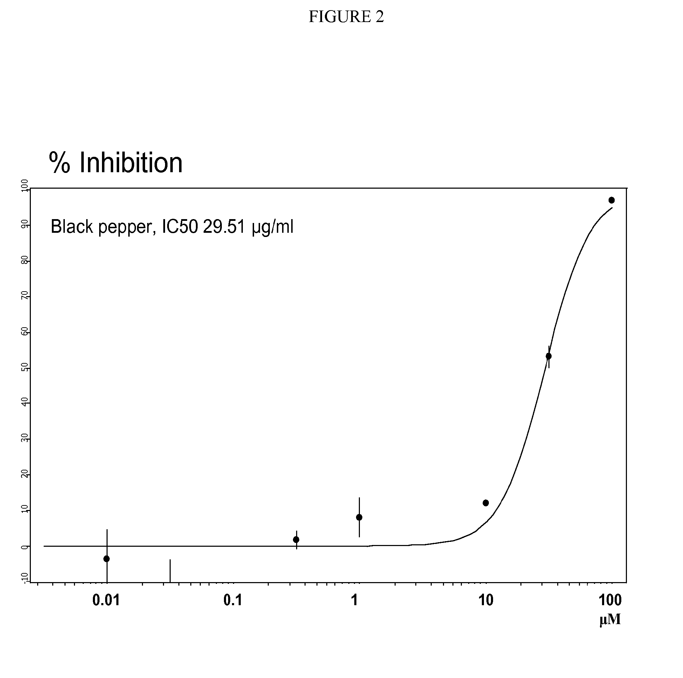

Glycine uptake via the GlyT1 transporter was inhibited by the addition of the black pepper extract or its major constituents in a dose-dependent manner. Sarcosine, ORG24598 and ALX5407 (all from Sigma, St. Louis, USA) were used as known inhibitors of GlyT1. The measured IC₅₀ values for inhibition of glycine uptake and representative dose-response curves are shown in Table 1. FIGURES 1 and 2 show the dose response curves for piperine and the extract.

Table 1: Measured IC₅₀ values for inhibition of glycine uptake into CHO cells by black pepper extract or its major constituents. Data is shown as mean ± s.e.m., where the IC₅₀ is stated as µM for pure compounds and as µg/mLfor extracts.

**Table 1**

| **Substance** | **IC₅₀ for tritiated glycine uptake** |
|---|---|
| SFCO2 black pepper extract | 32 µg/ml |
| Piperine (Aldrich, Cat. No.: P49007) | 48.7 µM |
| Piperlongumine (Sigma, cat. No.:P8588) | inactive |
| Piperlonguminine (Analyticon (Potsdam)) Cat. No P-0111-N-01 | inactive |
| Sarcosine (Sigma, Cat. no. S7672) | 35.9 µM |
| ORG24598 (Sigma, Cat. no. 07639) | 0.02 µM |
| ALX5407 (Sigma, Cat. no. A8977) | 6.46 nM |
| Gingko biloba (Huisong Pharmaceuticals, GB 102-070116) | inactive |

### Example 3

### Hippocampal slice cultures

Seven-day-old Wistar rats were decapitated using a guillotine. In less than 1 minute the skull was opened, the cerebral hemispheres were separated and transferred and both hippocampi were dissected and transferred into ice cold buffer containing 137mM NaCl, 5 mM KCl, 0.85 mM Na₂HPO₄, 1.5 mM CaCl₂, 0.66 mM KH₂PO₄, 0.28 mM MgSO₄, 1 mM MgCl₂, 2.7 mM NaHCO₃, 1 mM Kynurenic acid and 0.6% D-glucose.

Transversal hippocampal slices (400 µm) were prepared using a vibrating blade microtome (VT1200S; Leica Microsystems (Schweiz) AG, Heerbrugg, Switzerland) in the same buffer. Hippocampal slices were individually placed on a membrane insert (Millicell Culture Plate Inserts, 0.4 µm) and cultivated at 35 °C, 5% CO₂, 95% humidity in a medium containing a 1:1 mixture of BME and MEM (both from Invitrogen) containing 25% heat-inactivated horse serum, 1x GlutaMAX, 1x Penicillin/Streptomycin, 0.6% glucose and 1mM Kynurenic acid (Stoppini, Buchs and Muller (1991), J. Neurosci. Methods, 37(2), 173-82).

After 48 h in culture, synaptic NMDA receptors were activated by addition of black pepper extract or its constituents for 15 min in 140 mM NaCl, 5 mM KCl, 1.3 mM CaCl₂, 25 mM HEPES (pH 7.3), 33 mM D-glucose and 0.02 mM bicuculline methiodide. Sarcosine (100 µM) and ALX5407 (20 nM) were used routinely as positive controls. An additional positive control comprised the addition of 200 µM glycine to sister cultures. After the treatments, sections were washed and fixed for immunohistochemistry. Markers of enhanced synaptic activity, normally associated with long-term potentiation, representing an *ex vivo* model of learning and memory were quantitated (see Table 2, below).

Table 2. Relative activation of synaptic markers after treatment with black pepper extract or their constituent compounds (piperine, piperlongumine) in comparison to sister cultures treated with buffer. The activation of any of these markers (or a combination thereof) is observed in classical LTP experiments.

**TABLE 2**

| **Substance** | **pCREB** | **pMAPK** | **GluR1** |
|---|---|---|---|
| Black pepper SF CO₂ extract | -- | -- | 137-212 % |
| piperine | ± | ± | 119-1742% |
| piperlongumine | ± | ± | 412-931 % |
| Sarcosine | ± | ± | not done |
| Glycine (G7403) | ++ | ++ | 123-298% |
| Ginkgo biloba | ± | + | ± |

| | | | |
|---|---|---|---|
| ++++ shows a qualitative maximal activation, -- shows a reduced activation, whereas ++ signifies a half-maximum activation and ± demonstrated no change in immunoreactivity. | | | |

Treatment of hippocampal cultures with black pepper extract as well as with both piperine and piperlongumine induced some biochemical markers typical for LTP (pCREB: activated form of the cAMP response element binding protein; pMAPK: activated form of the mitogen-activated protein kinase; GLUR1: cell surface presence of AMPA receptor 1).

### Example 4

### Preparation of a soft gelatine capsule

A soft gelatine capsule may be prepared comprising the following ingredients:

| **Ingredient** | **Amount per Capsule** |
|---|---|
| Black pepper extract | 200 mg |
| Lecithin | 50 mg |
| Soy bean oil | 250 mg |

Two capsules per day for 3 months may be administered to a human adult. Cognitive functions, alertness and the ability to focus on work are seen to improve.

### Example 5

### Preparation of black pepper extract-coated potato crisps

Potatoes are sliced very thinly, drizzled with olive oil and baked in an oven (425 °F) until brown and crispy (about 30 minutes). Alternatively, the thin slices are deep fried in vegetable oil (350-375 °F) until crispy. Subsequently, the crisps are coated with a composition comprising sea salt and black pepper extract.

### Example 6

### Dry dog food containing piperine and/or piperlongumine or black pepper extract

A commercial basal diet for dogs (e.g. Mera Dog "Brocken", MERA-Tiernahrung GmbH, Marienstraße 80-84, D-47625 Kevelaer-Wetten, Germany) is sprayed with a suspension of corn oil containing piperine and/or piperlongumine or black pepper extract, together with antioxidants such as vitamin C (e.g. ROVIMIX® C-EC from DSM Nutritional Products Ltd, Kaiseraugst, Switzerland) and its derivatives, i.e. sodium ascorbyl monophosphate (e.g. STAY-C® 50 from DSM Nutritional Products Ltd, Kaiseraugst, Switzerland) or a mixture of tri-, di- and mono- phosphate esters of sodium/calcium L-ascorbate (e.g. ROVIMIX® STAY-C® 35 from DSM Nutritional Products Ltd, Kaiseraugst, Switzerland) in an amount sufficient to administer to a dog a daily dose of 10 mg piperine and/or piperlongumine or black pepper extract per kg body weight. The food composition is dried to contain dry matter of about 90% by weight. For an average dog of 10 kg body weight to consume approx. 200g dry feed per day, the dog food contains approx. 500 mg piperine and/or piperlongumine or black pepper extract per kg food. For heavier dogs, the feed mix is prepared accordingly.

For improvement of learning of young or adult dogs especially for training purposes, i.e. in hunting dog training, security, detection (drugs, detection of survivors in environmental catastrophes), protection or guidance dogs, the dog is fed daily with one portion per day.

For reduction of stress, fear and aggressiveness in dogs, the food can be given to dogs in animal shelter farms on a regular basis. Before veterinarian visits or stays in veterinarian clinics or holiday separation, the food is given at least one week before, during the stressful event and one week thereafter.

### Example 7

### Wet cat food containing piperine and/or piperlongumine or black pepper extract

A commercial basal diet for cats (e.g. Happy Cat "Adult", Tierfeinnahrung, Südliche Hauptstraße 38, D-86517 Wehringen, Germany) is mixed with a suspension of corn oil containing piperine and/or piperlongumine or black pepper extract, together with antioxidants such as vitamin C (e.g. ROVIMIX® C-EC from DSM Nutritional Products Ltd, Kaiseraugst, Switzerland) and its derivatives, i.e. sodium ascorbyl monophosphate (e.g. STAY-C® 50 from DSM Nutritional Products Ltd, Kaiseraugst, Switzerland) or a mixture of tri-, di- and mono- phosphate esters of sodium/calcium L-ascorbate (e.g. ROVIMIX® STAY-C® 35 from DSM Nutritional Products Ltd, Kaiseraugst, Switzerland) in an amount sufficient to administer to a cat a daily dose of 50 mg piperine and/or piperlongumine or black pepper extract per kg body weight.

For an average cat of 5 kg of body weight to consume approx. 400 g of wet food, the cat food contains 625 mg piperine and/or piperlongumine or black pepper extract per kg food. The food composition is dried to contain dry matter of about 90% by weight.

To foster general development in young cats, the food can be given to cats starting after weaning and continuing on a regular basis.

### Example 8

### Dog treats containing piperine and/or piperlongumine or black pepper extract

Commercial dog treats (e.g. Mera Dog "Biscuit" for dogs as supplied by Mera Tiernahrung GmbH, Marienstrasse 80-84, 47625 Kevelaer-Wetten, Germany) are sprayed with a suspension of corn oil containing piperine and/or piperlongumine or black pepper extract, together with antioxidants such as vitamin C (e.g. ROVIMIX® C-EC from DSM Nutritional Products Ltd, Kaiseraugst, Switzerland) and its derivatives, i.e. sodium ascorbyl monophosphate (e.g. STAY-C® 50 from DSM Nutritional Products Ltd, Kaiseraugst, Switzerland) or a mixture of tri-, di- and mono- phosphate esters of sodium/calcium L-ascorbate (e.g. ROVIMIX® STAY-C® 35 from DSM Nutritional Products Ltd, Kaiseraugst, Switzerland) in an amount sufficient to administer to the treats 5 - 25 mg piperine and/or piperlongumine or black pepper extract per g treat. The food composition is dried to contain dry matter of about 90% by weight.

The treat can be given before and during training as a reward up to 5 times per day.

### Example 9

### Cat treats containing piperine and/or piperlongumine or black pepper extract

Commercial cat treats (e.g. Whiskas Dentabits for cats as supplied by Whiskas, Masterfoods GmbH, Eitzer Str. 215, 27283 Verden/Aller, Germany) are sprayed with a suspension of corn oil containing piperine and/or piperlongumine or black pepper extract, together with antioxidants such as vitamin C (e.g. ROVIMIX® C-EC from DSM Nutritional Products Ltd, Kaiseraugst, Switzerland) and its derivatives, i.e. sodium ascorbyl monophosphate (e.g. STAY-C® 50 from DSM Nutritional Products Ltd, Kaiseraugst, Switzerland) or a mixture of tri-, di- and mono- phosphate esters of sodium/calcium L-ascorbate (e.g. ROVIMIX® STAY-C® 35 from DSM Nutritional Products Ltd, Kaiseraugst, Switzerland) in an amount sufficient to administer to the treats 1-10 mg piperine and/or piperlongumine or black pepper extract per g treats. The food composition is dried to contain dry matter of about 90% by weight.

To enhance learning and brain development a kitten can be given the treat as a reward up to 5 times per day.

### EXAMPLE 10

### Preparation of an enriched tomato juice

| **Ingredient** | **Amount [g]** |
|---|---|
| Black pepper Extract | 0.12 |
| **Tomato juice ad** | **1000** |

The juice contains ca. 30 mg black pepper extract per serving (250 ml).

### EXAMPLE 11

### Example 10

### Preparation of a broccoli soup

| **Ingredient** | **Amount** |
|---|---|
| Broccoli | 300 g |
| Onion powder | 14 g |
| Garlic powder | 5 g |
| Vegetable stock | 850 g |
| Potato flakes | 50 g |
| Powdered almonds | 20 g |
| Salt | 10 g |
| Citric acid | 0.5 g |
| Black pepper extract | 0.5 g |
| In total | 1250 g |

### Preparation:

The vegetable is cooked for 15-20 min, mixed and purreed with the vegetable stock. Then,

the other ingredients are added and heated again, before the soup is filled into tins.
1 serving (250 g) contains 100 mg of black pepper extract.

As a strengthener and for general well-being 2 servings per day (240 ml) is recommended.

## Claims

1. A method of improving cognitive function and/or psychosocial status in an animal or human comprising administering a cognitive function-improving or pyschosocial status improving amount of piperine and/or piperlongumine or a salt, derivative, metabolite or analogue and mixtures thereof, .

2. A method according to Claim 1 comprising administering piperine.

3. A method according to Claim 2 wherein the piperine is a component of a plant extract.

4. A method according to Claim 3 wherein the plant extract is a lipophilic black pepper extract.

5. A method according to Claim 1 comprising administering piperlongumine.

6. A method according to Claim 5 wherein piperlongumine is a component of a plant extract.

7. A method according to Claim 7 wherein the plant extract is a black pepper extract.

8. A method according to Claim 8 wherein the black pepper extract contains at least about 5 - 5% piperlongumine or piperine.

9. A method according to Claim 1 wherein the cognitive function and/or psychological status is selected from the group consisting of: maintaining cognitive wellness and balance, learning, language processing, problem solving, intellectual functioning, ability to cope with psychosocial burdens, attention and concentration, memory, the capacity for remembering, mental alertness, mental vigilance, mental fatigue, stabilization of mental status, a stress reliever, work-overload stress, stress-related exhaustion and/or bum out, and to promote relaxation.

10. A composition used in the manufacture of a nutraceutical or medicament for improving cognitive function or psycho-social status in an animal or human, which comprises a cognitive function-improving amount or psychosocial status improving amount of piperine and/or piperlongumine.

11. A composition according to Claim 10 comprising piperine.

12. A composition according to Claim 11 wherein the piperine is a component of a plant extract.

13. A composition according to Claim 10 which is a black pepper extract.

14. A composition according to Claim 10, wherein the composition comprises piperlongumine.

15. A composition according to Claim 14, wherein the piperlongumine is a component of a plant extract.

16. A composition according to Claim 16, which is a black pepper extract.

17. A composition according to Claim 10 wherein the cognitive function or psycho social status is selected from the group consisting of: maintaining cognitive wellness and balance, learning, language processing, problem solving, intellectual functioning, ability to cope with psychosocial burdens, attention and concentration, memory, the capacity for remembering, mental alertness, mental vigilance, mental fatigue, stabilization of mental status, a stress reliever, work-overload stress, stress-related exhaustion and/or bum out, and to promote relaxation.

18. A method according to Claim 1 comprising administering black pepper extract in an amount effective in inducing long-term potentiation.

19. A composition according to Claim 10, wherein the composition comprises black pepper in an amount effective in inducing long-term potentiation.
